# EUROPEAN PATENT APPLICATION

(11) **EP 4 768 569 A1**
(43) Date of publication of application: **01.07.2026**
(21) Application number: 24872929.5
(22) Date of filing: 25.09.2024
(51) Int. Cl.: C12N 1/14, C12N 15/01, C12P 13/02, C12R 1/645

(54) **WICKERHAMOMYCES SP. MICROORGANISM HAVING HIGH PRODUCTIVITY OF PHYTOSPHINGOSINE DERIVATIVE**

(30) Priority: 25.09.2023 KR 20230128563
(71) Applicant: Samyang KCI Corporation, Seosan-si, Chungcheongnam-do 31903 (KR)
(72) Inventor: AHN, Sinhye, Goyang-si, Gyeonggi-do 10374 (KR); LEE, Hansae, Suwon-si, Gyeonggi-do 16496 (KR); PARK, Busoo, Hanam-si, Gyeonggi-do 13014 (KR)
(74) Representative: AWA Sweden AB
(86) International application number: PCT/KR2024/014532
(87) International publication number: WO 2025/071221

(57) **Abstract**

The present disclosure relates to a microorganism having the ability to produce a phytosphingosine derivative and having resistance to cerulenin and/or EGTA, a composition including the same, and a method of producing a phytosphingosine derivative using the same.

## Description

### Technical Field

The present disclosure relates to a microorganism having high ability to produce a phytosphingosine derivative, such as tetraacetylphytosphingosine (TAPS) and/or triacetylphytosphingosine (TriAPS), a method of producing the microorganism, and a method of producing TAPS and TriAPS by using the microorganism. Specifically, the present disclosure relates to a *Wickerhamomyces* sp. microorganism having resistance to cerulenine and/or ethylene glycol-bis(3-aminoethyl ether)tetraacetic acid (EGTA), a composition including the microorganism, and a method of producing a phytosphingosine derivative by using the microorganism.

### Background Art

Ceramide is a type of sphingolipids with a structure in which fatty acids are linked to sphingosine. It accounts for about 40 % of the intercellular lipids of keratinocytes that make up the stratum corneum of the skin, and has the function of acting as a lipid barrier that prevents moisture evaporation and maintaining the orderly structure of the stratum corneum. The stratum corneum of the skin is composed of keratinized cells in a brick-like multi-layer structure, and since keratinized cells are firmly bound together by ceramides, cholesterol, and free fatty acids, it has been reported that applying a preparation containing ceramides to the skin helps with the skin barrier functions and skin hydration (see a thesis: A Comparative Study of the Skin Hydration Effect and Barrier Function of Natural Ceramide and Pseudo-ceramide, Kwon Minsu, 2005).

Ceramide can be produced from yeast and fungi, but among them, *Wickerhamomyces ciferrii* (also named *Pichia ciferrii)* is a yeast characterized by synthesizing tetraacetylphytosphingosine (TAPS), which is an acetylated derivative of phytosphingosine, from palmiotyl-CoA and the amino acid L-serine, and secreting TAPS outside the cell.

Phytosphingosine secreted by the *W. ciferrii* outside the cell is in a form of being substituted with an acetyl group, and depending on the number of substituted acetyl groups, TAPS, triacetylphytosphingosine, or diacetylphytosphingosine has been reported. TAPS is converted to phytosphingosine through a process of removing the acetyl groups, and the resulting phytosphingosine is industrially valuable because it can be used as a precursor required for ceramide synthesis.

In 1960, Wickerham, L.J. and Stodola, F.H.first discovered that the yeast Hansenula ciferrii NRRL 14091 secretes sphingolipids extracellularly and forms crystals around solid media. However, when cultured in liquid media, sphingolipids were barely produced (J.Bacteriol. 80: 484-491, 1960). On the other hand, the monoploid F-60-10 (NRRL 1301) isolated from the spores of the diploid 14091 was reported to secrete and produce significant amounts of sphingolipids even in liquid media.

*W. ciferrii* is a yeast that produces acetylated phytosphingosines, and TAPS is produced the most among the them. Studies have been conducted to increase the amount and productivity of acetylated phytosphingosines in strains through genetic engineering and metabolic engineering. For example, KR 10-0287483 discloses a technology for improving the productivity of TAPS by using a mutant strain of *P. ciferrii* into which a GAPDH-enhanced promoter has been inserted.

### Disclosure of Invention

### Technical Problem

The present disclosure provides a microorganism having the ability to produce a phytosphingosine derivative and having resistance to cerulenin.

The present disclosure provides a composition for producing a phytosphingosine derivative, the composition including at least one selected from the group consisting of the microorganism, a cell of the microorganism, a lysate of the cell of the microorganism, a culture of the microorganism, and an extract thereof.

The present disclosure provides a method of producing a phytosphingosine derivative, the method including culturing the microorganism.

### Solution to Problem

The present disclosure provides a *Wickerhamomyces* species (sp.) microorganism having the ability to produce a phytosphingosine derivative and having resistance to cerulenin.

The microorganism may further have resistance to ethylene glycol-bis(3-aminoethyl ether)tetraacetic acid (EGTA).

The microorganism may be *Wickerhamomyces ciferrii.*

The present disclosure provides a composition for producing a phytosphingosine derivative, the composition including at least one selected from the group consisting of the microorganism, a cell of the microorganism, a lysate of the cell of the microorganism, a culture of the microorganism, and an extract thereof.

The composition may further include at least one selected from the group consisting of cerulenin and EGTA, but is not limited thereto.

The present disclosure provides a method of producing a phytosphingosine derivative, the method including culturing the microorganism.

The present disclosure provides use of the microorganism having the ability to produce a phytosphingosine derivative and having resistance to cerulenin, for the production of a phytosphingosine derivative.

The present invention provides use of the microorganism having the ability to produce a phytosphingosine derivative and having resistance to cerulenin, for the preparation of a composition for producing a phytosphingosine derivative.

The microorganism provided by the present disclosure may have superior phytosphingosine productivity as compared to a wild-type microorganism (e.g., wild-type *W. ciferrii).*

Hereinafter, the present application will be described in more detail.

The present disclosure provides a *Wickerhamomyces* sp. microorganism having the ability to produce a phytosphingosine derivative and having resistance to cerulenin.

The phytosphingosine derivative may be at least one selected from the group consisting of monoacetylphytosphingosine, diacetylphytosphingosine, triacetylphytosphingosine (TriAPS), and tetraacetylphytosphingosine (TAPS), and specifically, may be TriAPS and/or TAPS.

The ability to produce a phytosphingosine derivative refers to the ability to have a high production amount of TAPS and/or TriAPS (e.g., a production amount per unit volume (L) of culture solution, mg/L) and/or a high production amount of TAPS and/or TriAPS per unit cell dry weight (e.g., a production amount per unit cell dry weight, mg/g-cell).

A production amount of the phytosphingosine derivative by the microorganism of the present disclosure, such as a production amount of TAPS and/or TriAPS production (e.g., a production amount per unit volume (L) of culture, mg/L) and/or a production amount of TAPS and/or TriAPS per unit cell dry weight (e.g., a production amount per unit cell dry weight, mg/g-cell), may be measured after culturing the microorganism under aerobic conditions at 30 °C for 96 hours, and specifically, may be measured by culturing the microorganism with shaking in a YMGL medium and extracting TAPS and/or TriAPS. However, embodiments are not limited thereto. The YMGL medium may contain glycerol, a yeast extract, a malt extract, and peptone.

The production amount of each type of phytosphingosine derivatives provided by the microorganism of the present disclosure may be confirmed by HPLC analysis after culturing and/or fermenting the microorganism and extracting phytosphingosine derivatives. However, embodiments are not limited thereto.

The production amount of TAPS by the microorganism may be, based on 100 % of the production amount of TAPS (mg/L) by wild-type *W. ciferrii,* 180 % or more, 200 % or more, 220 % or more, 240 % or more, 260 % or more, 280 % or more, 300 % or more, 320 % or more, for example, 326 %, but is not limited thereto.

The production amount of TAPS per unit cell dry weight (mg/g-cell) by the microorganism may be 15 mg or more, 20 mg or more, 25 mg or more, 30 mg or more, or 33 mg or more, for example, 35 mg. However, embodiments are not limited thereto.

The production amount of TAPS per unit cell dry weight (mg/g-cell) by the microorganism may be, based on 100 % of the production amount of TAPS per unit cell dry weight (mg/g-cell) by the wild-type *W. ciferrii,* 160 % or more, 180 % or more, 200 % or more, 220 % or more, 240 % or more, 260 % or more, 280 % or more, 300 % or more, 310 % or more, 315 % or more, or 318 % or more, for example, 318 %, but is not limited thereto. A unit of the production amount of TAPS per unit cell dry weight may be mg/g-cell, but is not limited thereto.

The total production amount (mg/L) of TAPS and TriAPS by the microorganism may be, based on 100 % of the total production amount of TAPS and TriAPS by the wild-type *W. ciferrii,* 150 % or more, 170 % or more, 190 % or more, 210 % or more, 230 % or more, 245 % or more, or 249 % or more, for example, 250 %, but is not limited thereto.

The total production amount of TAPS and TriAPS per unit cell dry weight (mg/g-cell) by the microorganism may be 80 mg or more, 90 mg or more, 95 mg or more, 100 mg or more, 105 mg or more, 110 mg or more, 115 mg or more, 120 mg or more, or 125 mg or more, for example, 127.1 mg, but is not limited thereto.

The total production amount of TAPS and TriAPS per unit cell dry weight (mg/g-cell) by the microorganism may be, based on 100% of the total production amount of TAPS and TriAPS per unit cell dry weight (mg/g-cell) by the wild-type *W. ciferrii,* 150 % or more, 180 % or more, 200 % or more, 210 % or more, or 240% or more, for example, 244%, but is not limited thereto. A unit of the production amount of TAPS per unit cell dry weight may be mg/g-cell, but is not limited thereto.

Among the phytosphingosine derivatives produced by the microorganism, including monoacetyl-, diacetyl-, triacetyl-, and tetraacetylphytosphingosine, the total production amount of TAPS and TriAPS may be higher than the production amount of each of the remaining types of phytosphingosine derivatives. For example, the production amount of TAPS may be the highest, and the production amount of TriAPS may be the second highest.

Among the phytosphingosine derivatives produced by the microorganism, including monoacetyl-, diacetyl-, triacetyl-, and tetraacetylphytosphingosine, the production ratio of TriAPS may be, based on the weight, 15 % or more, 18 % or more, 20 % or more, 21 % or more, 22 % or more, 23 % or more, 24 % or more, 15 % to 26 %, 18 % to 26 %, 20 % to 26 %, 21 % to 26 %, 22 % to 26 %, 23 % to 26 %, or 24 % to 26 %, for example, 24 % or 25 %, but is not limited thereto.

Among the phytosphingosine derivatives produced by the microorganism, including monoacetyl-, diacetyl-, triacetyl-, and tetraacetylphytosphingosine, the production ratio of TAPS may be, based on the weight, 65 % or more, 68 % or more, 70 % or more, 71 % or more, 72 % or more, 73 % or more, 65 % to 76 %, 68 % to 76 %, 70 % to 76 %, 71 % to 76 %, 72 % to 76 %, 73 % to 76 %, for example, 73 % or 75 %, but is not limited thereto.

The microorganism provided by the present disclosure has resistance to cerulenin and/or EGTA, which may act as toxins in the microbial growth and the production of phytosphingosine derivatives, and has excellent ability to produce a phytosphingosine derivative, specifically, excellent ability to produce TAPS and/or TriAPS.

The microorganism according to the present disclosure may have resistance to cerulenin.

Cerulenin is a fatty acid synthase inhibitor that, when added to a yeast growth medium, inhibits fatty acid synthesis and suppresses yeast growth. However, due to the mutation in the fatty acid synthesis pathway as described above, cerulenin may increase the ability to produce TAPS and TriAPS. Therefore, when cerulenin is used to increase the ability to produce TAPS and TriAPS, the microorganism used herein may have resistance to cerulenin along with the ability to produce a phytosphingosine derivative, becoming more desirable for increasing the ability to produce TAPS and TriAPS.

The resistance to cerulenin may refer to having the ability to grow and produce a phytosphingosine derivative, at a cerulenin concentration of 40 µg/ml or more in a medium containing the microorganism.

The microorganism according to the present disclosure may have resistance to EGTA.

The "ethylene glycol-bis(3-aminoethyl ether)tetraacetic acid (EGTA)" induces microbial mutations through enzyme chelation, and the resulting mutations may cause microorganisms to increase the ability to produce TAPS and TriAPS, and may be effectively used as an aminopolycarboxylic acid chelating agent in buffers that mimic the cellular environment.

The resistance to EGTA may include the ability to grow and produce a phytosphingosine derivative at an EGTA concentration of 30 µg/ml or more in a medium containing the microorganism.

The microorganism may be a *Wickerhamomyces* sp. strain, and specifically, may be *W. ciferrii, W.siamensis, W. alni, W.bisporus, W. silvicola, W. sydowiorum, W. chambardii, W. anomalus, W. arabprarous, W. canadensis, W. pijperi, W. strasburgensis, W. sydowiorum, W. bovis, W. chaumierensis, W. edaphicus, W. hampshirensis, W. jurtsmanii, W. Iynferdii, W. menglaensis, W. mori, W. mucosus, W. changensis, W. onychis, W. orientalis, W. patagonicus, W. queroliae, W. rabaulensis, W. scolytoplatypi, W. subpelliculosus, W. tratensis,* or *W. xylosica,* but is not limited thereto.

The microorganism may be *W. ciferrii* SYEC2-36, and may be a microorganism deposited to the Korea Culture Center of Microorganisms on February 22, 2023, with Accession No. KCCM13333P.

The deposited strain has at least one characteristic selected from the group consisting of (1) to (4):
(1) the production amount of TAPS per unit cell dry weight (mg/g-cell) by the microorganism of 15 mg or more;
(2) the total production amount of TAPS and TriAPS per unit cell dry weight (mg/g-cell) by the microorganism of 80 mg or more;
(3) resistance to cerulenin; and
(4) resistance to EGTA

The present disclosure may involve treating wild-type *W. ciferrii* with N-methyl-N'-nitro-N-nitrosoguanidine (NTG) and/or ultraviolet (UV) rays and identifying and selecting strains having better ability to produce TAPS and/or TriAPS than the parent strain of W. *ciferrii.* In addition, the microorganism may have resistance to one or more compounds selected from the group consisting of EGTA and cerulenin.

In an embodiment of the present disclosure, a composition for producing a phytosphingosine derivative may include at least one selected from the group consisting of a *Wickerhamomyces.* sp. microorganism, a cell of the microorganism, a lysate of the cell of the microorganism, a culture of the microorganism, and an extract thereof, wherein the microorganism has the ability to produce a phytosphingosine derivative and has resistance to cerulenin and/or EGTA. The composition may further include at least one selected from the group consisting of cerulenin and EGTA, but the embodiment is not limited thereto.

In an additional embodiment of the present disclosure, a method of producing a phytosphingosine derivative may include culturing at least one selected from a *Wickerhamomyces.* sp. microorganism, a cell of the microorganism, a lysate of the cell of the microorganism, a culture of the microorganism, and an extract thereof, wherein the microorganism has the ability to produce a phytosphingosine derivative and has resistance to cerulenin and/or EGTA.

Specifically, the culturing may be performed by culturing the *Wickerhamomyces.* sp. microorganism having the ability to produce a phytosphingosine derivative and having resistance to cerulenin. The method of producing the phytosphingosine derivative may further include, in addition to the culturing of the *Wickerhamomyces. sp.* microorganism, collecting a phytosphingosine derivative.

The culturing of the microorganism may include, although not limited thereto, culturing the microorganism in a medium for producing a phytosphingosine derivative, or culturing the microorganism in a seed culture medium, and/or culturing the microorganism in a medium for producing a phytosphingosine derivative.

When culturing in the seed culture medium, the temperature for the culturing may be 10 to 50 °C, 10 to 40 °C, 10 to 35 °C, 20 to 50 °C, 20 to 40 °C, 20 to 35 °C, 25 to 50 °C, 25 to 40 °C, or 25 to 35 °C, for example, 30 °C, but is not limited thereto.

When culturing in the seed culture medium, the time for the culturing may be 50 to 150 hours, 50 to 120 hours, 50 to 100 hours, 70 to 150 hours, 70 to 120 hours, 70 to 100 hours, 90 to 150 hours, 90 to 120 hours, 90 to 100 hours, 95 to 150 hours, 95 to 120 hours, or 95 to 100 hours, for example, 96 hours, but is not limited thereto.

In the seed culture medium, the culturing may be performed by shaking culture, and may be performed under various rpm conditions, for example, 100 to 500 rpm, 100 to 400 rpm, 100 to 300 rpm, 150 to 500 rpm, 150 to 400 rpm, 150 to 300 rpm, 200 to 500 rpm, 200 to 400 rpm, or 200 to 300 rpm, for example, 250 rpm, but is not limited thereto.

The seed culture medium may contain a carbon source, a nitrogen source, etc., and specifically may include at least one selected from the group consisting of a yeast extract, a malt extract, peptone, and glycerol, and may be, for example, a YMGL medium, but is not limited thereto.

The carbon source may be at least one selected from the group consisting of glycerol, malt, dextrin, glucose, sucrose, acetic acid, ethanol, molasses, and sulfite pulp waste liquid, which are commonly used for microbial culture, and specifically may be glycerol and/or malt, but is not limited thereto.

The nitrogen source may be at least one selected from the group consisting of nitrogen-containing organic substances, such as yeast, peptone, corn steep powder (CSP), corn steep liquor (CSL), urea, ammonia, ammonium sulfate, ammonium chloride, ammonium phosphate, and casein, and specifically may be yeast and/or peptone, but is not limited thereto.

The production medium for the phytosphingosine derivative may contain, although not limited thereto, at least one selected from the group consisting of glycerol, a yeast extract, CSP, ammonium sulfate, amino acids (e.g., serine, glycine, glutamic acid (monosodium glutamate)), sodium acetate, and calcium chloride.

When culturing in the production medium, the temperature for the culturing may be the same as or different from the temperature for the culturing in the seed culture, and specifically may be 10 to 50 °C, 10 to 40 °C, 10 to 35 °C, 20 to 50 °C, 20 to 40 °C, 20 to 35 °C, 25 to 50 °C, 25 to 40 °C, or 25 to 35 °C, for example, 30 °C, but is not limited thereto.

The culturing in the production medium may be performed under various rpm conditions, and for example, may be performed at 100 to 500 rpm, 100 to 400 rpm, 100 to 300 rpm, 100 to 200 rpm, 150 to 500 rpm, 150 to 400 rpm, 150 to 300 rpm or 150 to 200 rpm, for example, 180 rpm, but is not limited thereto.

When culturing in the production medium, the pH condition may be, although not limited thereto, 4 to 7, 4 to 6, 4 to 5.5, 4.5 to 7, 4.5 to 6, 4.5 to 5.5, 5 to 7, 5 to 6, 5 to 5.5, for example, 5.2.

The culturing in the production medium may be batch culture, fed-batch culture (e.g., fed-batch culture combined with continuous fermentation), or continuous culture, and the culture may be performed in one or more of these types, but is not limited thereto. The fed-batch culture combined with continuous fermentation refers to continuous culture or culture with adding a medium during the culture process.

The culturing in the production medium may be performed by fed-batch culture combined with continuous fermentation, but is not limited thereto.

The culturing in the production medium by fed-batch culture combined with continuous fermentation may involve inoculating the microorganism into the production medium while supplying an additional medium. Specifically, the additional medium may be added after all glycerol in the production medium has been depleted, thereby supplying glycerol. The "additional medium" refers to a medium that is intermittently or continuously supplied to the culture medium after initiation of the microbial culture, in addition to the initial culture medium.

When culturing by fed-batch culture combined with continuous fermentation, the production medium may contain, although not limited thereto, at least one selected from the group consisting of glycerol, a yeast extract, CSP, amino acids (e.g., serine, glycine, glutamic acid (monosodium glutamate)), ammonium sulfate, and calcium chloride (e.g., calcium chloride dihydrate).

The additional medium may contain, although not limited thereto, at least one selected from the group consisting of glycerol, a yeast extract, amino acids (e.g., serine, glycine, and/or glutamic acid (monosodium glutamate)), ammonium sulfate, calcium chloride (e.g., calcium chloride dihydrate), and sodium acetate.

The additional medium may be added at a concentration of 1 to 10 g/L, 1 to 8 g/L, 1 to 6 g/L, 1 to 5 g/L, 3 to 10 g/L, 3 to 8 g/L, 3 to 6 g/L, or 3 to 5 g/L, for example, 4 g/L, per hour, but is not limited thereto.

When culturing by fed-batch culture combined with continuous fermentation, CSP may be additionally supplied when the O.D value of the production medium may be 100 to 150, 100 to 140, 110 to 150, 110 to 140, 120 to 150, or 120 to 140, for example, 130, and/or 230 to 300, 230 to 280, 230 to 270, 240 to 300, 240 to 280, 240 to 270, 250 to 300, 250 to 280, or 250 to 270, for example, 260.

The O.D value may be measured for light having a wavelength of 400 to 800 nm, 400 to 700 nm, 400 to 650 nm, 400 to 620 nm, 500 to 800 nm, 500 to 700 nm, 500 to 650 nm, 500 to 620 nm, 550 to 800 nm, 550 to 700 nm, 550 to 650 nm, 550 to 620 nm, 580 to 800 nm, 580 to 700 nm, 580 to 650 nm, or 580 to 620 nm, for example, 600 nm, but is not limited thereto.

The CSP may be supplied such that the concentration of the CSP in the production medium is 1 to 5 g/L, 1 to 4 g/L, 2 to 5 g/L or 2 to 4 g/L, for example, 3 g/L, but is not limited thereto.

The CSP may be supplied as being dissolved in distilled water, and for example, may be supplied as being dissolved in primary distilled water, secondary distilled water, and/or tertiary distilled water, but is not limited thereto.

When sodium acetate is added to the production medium during the culturing in the production medium by fed-batch culture combined with continuous fermentation, the pH in the production medium increases, which may make initial culturing difficult. However, when sodium acetate contained in the additional medium is added after glycerol is completely depleted following the initial culture, the growth of cells and/or the production amount of phytosphingosine of the cells (e.g., monoacetylphytosphingosine, diacetylphytosphingosine, TriAPS, and/or TAPS) may be increased.

When culturing in the production medium by fed-batch culture combined with continuous fermentation, the temperature may be 10 to 50 °C, 10 to 40 °C, 10 to 35 °C, 20 to 50 °C, 20 to 40 °C, 20 to 35 °C, 25 to 50 °C, 25 to 40 °C, or 25 to 35 °C, for example, 30 °C, but is not limited thereto.

The culturing in the production medium by fed-batch culture combined with continuous fermentation may be performed under various rpm conditions, and for example, may be performed 500 to 1,500 rpm, 700 to 1,500 rpm, 800 to 1,500 rpm, 850 to 1,500 rpm, 500 to 1,200 rpm, 700 to 1,200 rpm, 800 to 1,200 rpm, 850 to 1,200 rpm, 500 to 1,000 rpm, 700 to 1,000 rpm, 800 to 1,000 rpm, 850 to 1,000 rpm, 500 to 950 rpm, 700 to 950 rpm, 800 to 950 rpm, or 850 to 950 rpm, for example, 900 rpm, but is not limited thereto.

When culturing in the production medium by fed-batch culture combined with continuous fermentation, the pH condition may be 4 to 7, 4 to 6, 4 to 5.5, 4.5 to 7, 4.5 to 6, 4.5 to 5.5, 5 to 7, 5 to 6, 5 to 5.5, for example, 5.2, but is not limited thereto.

The culturing in the production medium by fed-batch culture combined with continuous fermentation may be performed under conditions of 0.1 to 10 vvm, 0.1 to 8 vvm, 0.1 to 6 vvm, 0.1 to 4 vvm, 0.1 to 2 vvm, 0.1 to 1.5 vvm, 0.1 to 1.2 vvm, 0.5 to 10 vvm, 0.5 to 8 vvm, 0.5 to 6 vvm, 0.5 to 4 vvm, 0.5 to 2 vvm, 0.5 to 1.5 vvm, 0.5 to 1.2 vvm, 0.8 to 10 vvm, 0.8 to 8 vvm, 0.8 to 6 vvm, 0.8 to 4 vvm, 0.8 to 2 vvm, 0.8 to 1.5 vvm, or 0.8 to 1.2 vvm, for example, 1 vvm, but is not limited thereto.

The collecting of the phytosphingosine derivative may involve conventional extraction and collection techniques to collect the microorganism (cell) provided by the present disclosure and/or a fermentation broth (e.g., a culture broth of the microorganism provided by the present invention). Specifically, the cultured cell and/or a fermentation supernatant may be collected. Once the desired product is obtained, it may be used directly or undergo another process depending on the intended use. For example, acetylated derivatives of sphingosine, dihydrosphingosine, and/or phytosphingosine may be deacetylated enzymatically or chemically.

The phytosphingosine derivative obtained according to the present disclosure may be used in food, cosmetics, and skin care compositions, specifically in a ceramide form for food applications in the form of glycosphinogolipids.

### Advantageous Effects of Disclosure

The microorganism having resistance to cerulenin and/or EGTA according to the present disclosure has the excellent ability to produce phytosphingosine derivatives. When producing phytosphingosine derivatives by using the microorganism, phytosphingosine may be produced with high efficiency and in an eco-friendly manner, as compared to phytosphingosine produced by a chemical synthesis method.

### Mode for the Invention

The present disclosure will be described in more detail with reference to the following examples, but the scope of the present disclosure is not intended to be limited to the following examples.

### Example 1. Production of mutant microorganisms with improved production ability of TAPS (1)

### Example 1-1: Culturing and collecting of parent strain

A wild-type strain of *W. ciferrii* was mutated to obtain a mutant microorganism having high productivity of TAPS. Specifically, the strain ATCC 14091 was obtained as the wild-type strain of *W. ciferrii.*

50 µl of glycerol stock was mixed with 3 ml of YMGL broth having a pH of 5.5 and components as shown in Table 1, and the mixture was cultured with shaking at 30 °C for 24 hours. The glycerol stock is a 1:1 mixture of a wild-type strain culture medium and a 40 (w/w)% glycerol solution, stored at -70 °C.

**[Table 1]**

| Components | Concentration (g/L) |
|---|---|
| Yeast extract | 3 |
| Malt extract | 3 |
| Peptone | 5 |
| Glycerol | 30 |

After the shaking culture, 50 µl of the culture was taken and inoculated into 3 ml of new YMGL broth and cultured at 30 °C for 12 hours. The cell concentration was confirmed by measuring the absorbance at 600 nm in the culture solution, and the cells were harvested when the OD value reached 2. Specifically, the culture solution was centrifuged (12,000 rpm, 10 minutes) to collect only the cells, and then washed twice with 50 mM citrate buffer to remove the medium components.

### Example 1-2: Strain mutation treatment

The washed cells were treated with N-methyl-N'-nitro-N-nitrosoguanidine (NTG) at a concentration of 0.05 mg/ml, and then reacted at room temperature for 30 minutes to produce mutagenic microbial cells. The mutagenic microbial cells were diluted with sterile distilled water, and ethylene glycol-bis(3-aminoethyl ether)tetraacetic acid (EGTA), which is a mutation marker, was added thereto at a concentration range of 2 to 50 µg/ml to check the degree of growth inhibition. The growth inhibition was observed at concentrations starting from a concentration of 30 µg/ml such that the microbial cells diluted with the sterile distilled water were spread onto an YNB (w/o amino acid) plate medium containing EGTA at a concentration of 30 µg/ml.

From the plate medium onto which the cells were spread, a mutant library was obtained by exposing the plate medium to UV light having a wavelength of 254 nm for 20 seconds at a 15 cm interval, followed by culturing. The mutagenic microorganisms are microorganisms having high ability to produce TAPS and having resistance to EGTA, and each microorganism was given a random number and named in the form of E and number (e.g., E284).

### Example 1-3: Primary colony selection and culture

To select a microorganism having excellent ability to produce TAPS among the mutagenic microorganisms of Example 1-2, single colonies that are different in shape and size and did not grow overlapping with other colonies were selected from among the colonies grown on the plate medium of Example 1-2.

The selected colonies and the parent strain that has not undergone the mutation treatment were separately inoculated into a test tube containing 3 ml of the YMGL broth as shown in Table 1. Each test tube was then cultured with shaking at 250 rpm for 96 hours at a temperature of 30 °C. 100 % methanol at 9 times the volume of the culture solution was added and vigorously stirred using a finemixer at room temperature for 60 minutes to extract TAPS.

Afterwards, the supernatant obtained by centrifugation at 12,000 rpm for 10 minutes was filtered through a 0.2 µm filter, and the production amount of TAPS was determined by HPLC analysis. The results are shown in Table 2.

Specific HPLC analysis was performed by using HPLC-UV equipped with a ZORBAX SB-C8 column (Agilent, 4.6 mm x 150 mm, 3.5 µm, USA). For the TAPS quantitative calculation, TAPS was purchased from Sigma-Aldrich to obtain a quantitative curve, and the production amount of TAPS (TAPS titer (mg/L)) and the yield of TAPS (mg/g-cell) per unit cell weight were measured.

**[Table 2]**

| Microorganism | TAPS amount (mg/L) | TAPS yield (mg/g-cell) |
|---|---|---|
| Wild-type | 162 | 11.1 |
| E19 | 393 | 31.0 |
| E28 | 398 | 22.1 |
| E125 | 315 | 19.9 |
| E201 | 388 | 38.3 |
| E218 | 406 | 26.8 |
| E284 | 528 | 36.2 |

As a result of measuring the production amount of TAPS by the HPLC analysis, the production amount of TAPS by the wild-type strain was measured to be 162 mg/mL, and six mutant microorganisms (E284, E218, E201, E125, E28, and E19) of which the production amount of TAPS was higher (393 to 528 mg/L) than the wild-type strain were selected. Among the six selected microorganisms, the E284 mutant strain showed the highest production amount of TAPS (528 mg/L).

### Example 2. Production of mutant microorganisms with improved production ability of TAPS (2)

Among the six strains having the increased production amount of TAPS compared to the wild-type strain, secondary mutation was performed by using the E284 strain that showed a significant increase in the production amount and yield of TAPS. Selection of mutant strains was performed substantially the same as the methods of Examples 1-1 and 1-2. Here, the mutagen-treated strains were observed to check the degree of growth inhibition by adding cerulenin as a marker, instead of EGTA, at a concentration range of 0 to 50 µg/ml. The inhibition was observed at concentrations starting from 40 µg/ml such that the mutagen-treated strains were spread onto a plate medium containing 40 µg/ml of cerulenin to discover secondary mutant strains.

The additional mutagenic microorganisms are microorganisms that were additionally selected with cerulenin from among the primary screened microorganisms having resistance to EGTA, and each microorganism was given a random number and named in the form of EC number-number (e.g., EC2-34).

For the additional mutagenic microorganisms, the production amount of TAPS and the production amount of TAPS per unit cell dry weight (production yield) were confirmed through TAPS extraction and HPLC analysis in substantially the same manner as in Example 1-3. Likewise, for the E284 of Example 1-3 and the wild-type strain of Example 1, TAPS extraction, production amount of TAPS, and production yield of TAPS were re-confirmed in the same manner, and the results are shown in Table 3.

**[Table 3]**

| Microorganism | TAPS amount (mg/L) | TAPS yield (mg/g-cell) |
|---|---|---|
| Wild-type | 162 | 11 |
| E284 | 393 | 31 |
| EC1-4 | 654 | 45 |
| EC2-34 | 705 | 46 |
| EC2-36 | 528.2 | 35 |
| EC3-14 | 540 | 37 |
| EC4-43 | 649 | 44 |

As a result of measuring the production amount of TAPS through the HPLC analysis, the production amount of TAPS and the production yield of TAPS by the wild-type strain were measured to be 162 mg/L and 11 mg/g-cell, respectively, whereas the production amount of TAPS and the production yield of TAPS by the primary selected E284 strain were measured to be 393 mg/L and 31 mg/g-cell, respectively.

Among the mutant strains selected in the medium supplemented with cerulenin, five microorganisms (EC1-4, EC2-34, EC2-36, EC3-14, and EC4-43) were selected that had increased production amount of TAPS (528 to 705 mg/L) and increased production yield of TAPS (35 to 46 mg/g-cell), as compared to the wild-type strain and the E284 strain.

### Example 3: Confirmation of production amount of TAPS/acetylated phytosphingosine in flask

### Example 3-1: Strain culture

To compare the wild-type strain and the mutant *W. ciferrii* strain in terms of the production amount of TAPS, flask-based culture was performed thereon. Five mutant strains having resistance to both EGTA and cerulenin and the wild-type strain were cultured in flasks to confirm the production amounts of TAPS and TriAPS.

Specifically, 3 ml of the YMGL broth of Table 1 was dispensed into a 15 ml test tube. Then, inoculating with the wild-type strain and inoculating with colonies of the five mutant strains having resistance to both EGTA and cerulenin obtained in Example 2 were performed, followed by culturing with shaking at 250 rpm for 24 hours at 30 °C, thereby obtaining a seed culture solution.

The seed culture solution was inoculated into a 250 ml baffled flask containing 30 ml of a production medium having the components shown in Table 4 such that the OD value of the culture solution became 0.3. Then, the culture solution was cultured with shaking at 30 °C for 96 hours at 180 rpm, thereby obtaining a production culture. The pH of the production medium was maintained at pH 5.2 by adding 50 mM MES buffer.

**[Table 4]**

| Component | Concentration (g/L) |
|---|---|
| Glycerol | 100 |
| Yeast extract | 0.7 |
| Corn steep powder | 3 |
| (NH4)₂SO₄ | 1 |
| Sodium acetate | 2 |
| CaCl₂.2H₂O | 1 |
| Serine | 1 |
| pH 5.2 | |

### Example 3-2: Analysis of production amounts of TriAPS and TAPS

For the production culture solution obtained in Example 3-1, 100 % methanol was added to the culture solution in substantially the same manner as in Example 1-3, and the supernatant obtained by centrifugation was separated and purified to obtain an analysis sample.

For the analysis samples, the total production amount of TAPS and TriAPS (Tri + TAPS titer (mg/L)) and the production amount per unit cell dry weight (production yield) (Tri + TAPS yield (mg/g-cell)) were confirmed through HPLC analysis in substantially the same manner as in Examples 1-3.

Specifically, the freeze-dried powder of the analysis sample was dissolved in CDCl₃, and the structure of the analysis sample was finally confirmed through NMR analysis by using Bruker Avance DPX 400 (400 MHz) to confirm the presence of TriAPS in the sample. Separately, the analysis sample was separated, purified, and concentrated by using recycling preparative HPLC (manufacturer: Jai, Japan Analytical Industry Co., LTD./ Resin: C18) to obtain freeze-dried powder, and HPLC analysis was performed in substantially the same manner as in Example 1-3 to measure the production amount of TriAPS and the production yield of TriAPS per unit cell weight. The total production amount of TAPS and TriAPS and the total production yield of TAPS and TriAPS per unit cell weight, obtained through the HPLC analysis, are shown in Table 5.

**[Table 5]**

| Microorganism | Total amount of TAPS + TriAPS (mg/L) | Total yield of TAPS + TriAPS (mg/g-cell) |
|---|---|---|
| Wild-type strain | 1257.0 | 52.0 |
| E284 | 2759.7 | 97.6 |
| EC1-4 | 1926.5 | 82.6 |
| EC2-34 | 3079.3 | 97.7 |
| EC2-36 | 3152.4 | 127.1 |
| EC3-14 | 3234.7 | 112.0 |
| EC4-43 | 2387.7 | 100.2 |

As a result of measuring the total production amount and the total production yield of TAPS and TriAPS, two strains were finally identified: the EC3-14 strain showing the highest increase in the total production amount of TriAPS and TAPS as compared to the wild-type strain; and the EC2-36 strain showing the highest total yield of TAPS and TriAPS per unit cell. The two selected strains, i.e., the EC2-36 strain and the EC3-14 strain, were cultured in a fermenter to finally confirm the total production amount of TAPS and acetylated phytosphingosine.

### Example 4: Production of acetylated phytosphingosine in a fermenter

To finally confirm the total production amount of TAPS and acetylated phytosphingosine by culturing the two selected strains in Example 3-2, i.e., the EC2-36 strain and the EC3-14 strain, in a fermenter, fed-batch culture combined with continuous fermentation was performed.

Specifically, microbial colonies of each of the EC2-36 strain and the EC3-14 strain were obtained from the YMGL agar medium of Example 1 prepared by adding 15 g/L of agar to the YMGL broth. 3 ml of the YMGL broth of Table 1 was dispensed into a 15 ml test tube. Then, inoculating with colonies of each of the EC2-36 strain and the EC3-14 strain was performed, followed by culturing with shaking at 250 rpm for 24 hours at 30 °C, thereby obtaining a seed culture solution.

The seed culture solution was inoculated into a 500 ml baffled flask containing 100 ml of the YMGL broth of Table 1 such that the OD value of the culture solution became 0.3. Then, the culture solution was cultured with shaking at 30 °C for 24 hours at 180 rpm, thereby obtaining a preculture.

The preculture was inoculated into a 5 L jar fermenter such that the OD value of the fermentation broth became 0.3. Here, 2 L of a production medium containing the components of Table 6 was used as the culture medium, and when 20 to 30 g/L of glycerol remained in the production medium, the culturing was performed while feeding 4 g/L of glycerol per hour by using a feeding solution containing the components of Table 7.

**[Table 6]**

| Component | Concentration (g/L) |
|---|---|
| Glycerol | 100 |
| Yeast extract | 0.7 |
| Corn steep powder | 3 |
| Serine | 1 |
| Glycine | 1 |
| Monosodium glutamate | 1.27 |
| Ammonium sulfate | 1 |
| Calcium chloride dihydrate | 1 |

**[Table 7]**

| Component | Concentration (g/L) |
|---|---|
| Glycerol | 1,000 |
| Yeast extract | 7 |
| Serine | 5 |
| Glycine | 5 |
| Monosodium glutamate | 6.3 |
| Ammonium sulfate | 5 |
| Calcium chloride dihydrate | 5 |
| Sodium acetate | 20 |

A sterilized solution, prepared by mixing corn steep powder (CSP) with 30 mL of DDW such that the concentration of CSP became 3 g/L in the culture solution, was added when the OD values of the culture solution under light with a wavelength of 600 nm were 130 and 260, respectively.

Filtered air was supplied to the production medium at 2 L/min such that the air flow rate became 1 vvm, and the production medium was stirred at 900 rpm under temperature conditions of 30°C. The medium pH was adjusted to 5.2 by using 9 % NH₄OH, and antifoam (SB2121, Struktol) in undiluted form was added at 10 µl/20 min, and the fermentation was performed for 151 hours.

100 % methanol was added to the previously obtained culture solution in substantially the same manner as in Example 3-2, and phytosphingosine and derivatives thereof were extracted by centrifugation. The production amount of each type of acetylated phytosphingosine was analyzed, and the results of analyzing the product yield of the EC2-36 strain and the EC3-14 strain are shown in Table 8.

**[Table 8]**

| Type of phytosphingosine | Production amount of EC2-36 (g/L) | Production amount of EC3-14 (g/L) |
|---|---|---|
| Monoacetylphytosphingosine | 0.01 | 0.02 |
| Diacetylphytosphingosine | 0.11 | 0.14 |
| Triacetylphytosphingosine (TriAPS) | 4.19 | 2.76 |
| Tetraacetylphytosphingosine (TAPS) | 13 | 8.1 |
| Total | 17.31 | 11.02 |

As a result of analyzing the products, TriAPS and TAPS showed the highest production amount of acetylated phytosphingosine by the EC2-36 microorganism, specifically, 4.19 g/L for TriAPS and 13 g/L of TAPS. TriAPS and TAPS showed the highest production amount of acetylated phytosphingosine by the EC3-14 strain, specifically, 2.76 g/L for TriAPS and 8.1 g/L of TAPS.

The EC2-36 strain, which was confirmed to have the high ability to produce TriAPS and TAPS, was deposited to the Korea Culture Center of Microorganisms on February 22, 2023, with Accession No. KCCM13333P, and was named SYEC2-36.

### [Accession Number]

Name of depository: Korea Culture Center of Microorganisms
Accession No: KCCM13333P
Date of deposit: 20230222

## Claims

1. A *Wickerhamomyces* species (sp.) microorganism having the ability to produce a phytosphingosine derivative and having resistance to cerulenin.

2. The *Wickerhamomyces* sp. microorganism of claim 1, wherein the phytosphingosine derivative comprises at least one selected from the group consisting of tetraacetylphytosphingosine (TAPS) and triacetylphytosphingosine (TriAPS).

3. The microorganism of claim 2, wherein a production amount of TAPS per unit dry cell weight (mg/g-cell) by the microorganism is 160 % or more based on 100 % of a production amount per unit dry cell weight by wild-type *Wickerhamomyces ciferrii.*

4. The microorganism of claim 2, wherein a total production amount of TAPS and TriAPS per unit dry cell weight (mg/g-cell) by the microorganism is 150 % or more based on 100 % of a production amount per unit dry cell weight by wild-type *Wickerhamomyces ciferrii.*

5. The microorganism of claim 1, wherein the resistance to cerulenin is to have the ability to grow and produce a phytosphingosine derivative, at a cerulenin concentration of 40 µg/ml or more in a medium including the microorganism.

6. The microorganism of claim 1, wherein the microorganism further has resistance to ethylene glycol-bis(3-aminoethyl ether)tetraacetic acid (EGTA).

7. The microorganism of claim 6, wherein the resistance to EGTA is to have the ability to grow and produce a phytosphingosine derivative at an EGTA concentration of 30 µg/ml or more in a medium including the microorganism.

8. The microorganism of claim 1, wherein the microorganism is *Wickerhamomyces ciferrii.*

9. The microorganism of claim 1, wherein the microorganism is *Wickerhamomyces ciferrii* having Accession No. KCCM13333P.

10. A composition for producing a phytosphingosine derivative, comprising at least one selected from the group consisting of a cell of the *Wickerhamomyces* sp. microorganism of any one of claims 1 to 9, a lysate of the cell of the microorganism, a culture of the microorganism, and an extract thereof.

11. The composition for producing a phytosphingosine derivative of claim 10, further comprising at least one selected from the group consisting of cerulenin and ethylene glycol-bis(3-aminoethyl ether)tetraacetic acid (EGTA).

12. A method of producing a phytosphingosine derivative, the method comprising: culturing the *Wickerhamomyces* sp. microorganism according to any one of claims 1 to 9; and collecting a phytosphingosine derivative from a culture solution of the microorganism.

13. The method of producing a phytosphingosine derivative of claim 12, wherein the culturing of the microorganism is performed by fed-batch culture combined with continuous fermentation.

14. The method of producing a phytosphingosine derivative of claim 13, wherein the culturing of the microorganism is to inoculate the microorganism in a production medium and supply an additional medium while culturing the microorganism.

15. The method of producing a phytosphingosine derivative of claim 14, wherein the culturing of the microorganism is performed under a pH condition of 4 to 7.

16. The method of producing a phytosphingosine derivative of claim 14, wherein the production medium comprises at least one selected from the group consisting of glycerol, a yeast extract, corn steep powder, an amino acid, ammonium sulfate, and calcium chloride.

17. The method of producing a phytosphingosine derivative of claim 14, wherein the medium additionally supplied is added at a concentration of 1 to 10 g/L per hour.

18. The method of producing a phytosphingosine derivative of claim 14, wherein the medium additionally supplied comprises at least one selected from the group consisting of glycerol, a yeast extract, an amino acid, ammonium sulfate, calcium chloride, and sodium acetate.

19. The method of producing a phytosphingosine derivative of claim 14, wherein corn steep powder is additionally supplied when an optical density (O.D) value of the production medium is 100 to 150 or 230 to 300.

20. The method of producing a phytosphingosine derivative of claim 19, wherein the corn steep powder is supplied such that a concentration of the corn steep powder in the production medium is 1 to 5 g/L.

21. The method of producing a phytosphingosine derivative of claim 14, wherein the culturing is performed under temperature conditions of 10 to 50 °C.
